# EUROPEAN PATENT SPECIFICATION

(11) **EP 2 285 429 B1**
(45) Date of publication and mention of the grant of the patent: **02.05.2018**
(21) Application number: 09759018.6
(22) Date of filing: 22.05.2009
(51) Int. Cl.: A61L 31/10, A61L 31/14, A61L 31/16

(54) **REDUCING BIOABSORPTION TIME OF POLYMER COATED IMPLANTABLE MEDICAL DEVICES USING POLYMER BLENDS**
REDUZIERUNG DER BIOABSORPTIONSZEIT POLYMERBESCHICHTETER IMPLANTIERBARER MEDIZINISCHER VORRICHTUNGEN MITHILFE VON POLYMERMISCHUNGEN
RÉDUCTION DU TEMPS DE BIOABSORPTION DE DISPOSITIFS MÉDICAUX IMPLANTABLES REVÊTUS DE POLYMÈRE À L'AIDE DE MÉLANGES DE POLYMÈRE

(30) Priority: 02.06.2008 US 131623
(43) Date of publication of application: 23.02.2011
(73) Proprietor: Medtronic Vascular Inc., Santa Rosa, CA 95403 (US)
(72) Inventor: CHENG, Peiwen, Santa Rosa CA 95409 (US); JIANG, Kevin, Santa Rosa CA 95409 (US)
(74) Representative: Zimmermann & Partner Patentanwälte mbB
(86) International application number: PCT/US2009/044961
(87) International publication number: WO 2009/148857

(56) References cited:
- US-A1- 2004 224 001
- US-A1- 2005 112 170
- US-A1- 2006 074 191
- US-A1- 2007 288 088
- SHARKAWI T ET AL: "evaluation of the in vitro drug release from resorbable biocompatible coatings for vascular stents" JOURNAL OF BIOACTIVE AND COMPATIBLE POLYMERS, SAGE PUBLICATIONS, GB LNKD- DOI:10.1177/0883911505051661, vol. 20, no. 2, 1 January 2005 (2005-01-01), pages 153-168, XP008099662 ISSN: 0883-9115

## Description

### FIELD OF THE INVENTION

Described herein are polymer blends useful in reducing bioabsorbtion time by blending different weight polymers. This blending will reduce a conventional polymer's bioabsorption time and/or allow tailoring of bioabsorption times.

### BACKGROUND OF THE INVENTION

Drug eluting stents (DES) have been on the market for several years now with excellent clinical success. Drug eluting stents have revolutionized the vascular and cardiologic medicine, aiding in such complications as vulnerable plaque rupture, stenosis, restenosis, ischemic myocardial infarct, and atherosclerosis. However, as with any evolving technology, there have been drawbacks.

One major drawback of DES is late thrombosis. Late thrombosis is thought to occur due to incomplete endothelial cell migration to the DES surface. The thrombosis may be due to the polymers used to coat the DES or the drugs eluted by the DES.

Commonly, patients suffering from DES-induced late thrombosis are forced to take anticoagulant (anti-clotting) or anti-platelet drugs such as clopidogrel or acetylsalicylic acid for longer times than normal. The longer systemic administration of drugs of these types can be harmful to the patient.

One method to remedy this problem is to introduce a pro-healing drug into the DES. However, it is difficult to find a pro-healing drug that prohibits smooth muscle cell proliferation while promoting endothelial cell growth. Another method entails the use of a non-polymer coated DES. However, this approach is difficult because it adds complexity to the structural design of the DES. In addition, the controlled release of a drug is not attainable as there is no presence of a polymer coating to allow for controlled release. US 2005/0112170 describes coatings for implantable devices comprising polymers of lactic acid and methods for fabricating the same. US 2006/0074191 describes blends of poly(ester amide) polymers.

Sharkawi et al. (Journal of Bioactive and Compatible Polymers, Vol. 20, No.2, 2005, pages 153-168), according to its title, relates to the evaluation of the in vitro drug release from resorbable biocompatible coatings for vascular stents.

US 2007/0288088, according to its title, relates to drug eluting stents with a biodegradable release layer attached with an electro-grafted primer coating.

Methods need to be developed to remedy the problems associated with DES-induced late thrombosis. Described herein is a system and method for DES therapy with a potential for reduced instance of late thrombosis.

### SUMMARY OF THE INVENTION

Described herein is a polymer system for reducing the risk of late thrombosis associated with implantable medical devices. The polymer system can be used to coat implantable medical devices. The polymer system can be fine tuned to degrade in a predictable amount of time. The polymer system can further be impregnated with one or more bioactive agents. These bioactive agents can diffuse out of the polymer into the surrounding tissue or can be released as the polymer is degraded.

Described herein is a medical device comprising: (a) a stent comprising a non-erodable metal; (b) a bioabsorbable polymer system coated on at least a portion of said metal; said polymer system comprising a blend of a least two polymers having different weight average molecular weights; wherein the ratio of weight average molecular weights is 1:2 to 1:10 and provides a pre-selected bioabsorption time; and (c) at least one bioactive agent dispersed in at least a portion of said polymer coating. In one embodiment, the stent is selected from the group consisting of woven stents, individual ring stents, sequential ring stents, closed cell stents, open cell stents, laser cut tube stents, ratchet stents, and modular stents. In another embodiment, the metal is selected from the group consisting of stainless steel, tantalum, titanium, nickel-titanium alloys, shape memory alloys, super elastic alloys, low-modulus Ti-Nb-Zr alloys, cobalt-nickel alloy steel (MP-35N), and combinations thereof.

In one embodiment, the polymers are selected from the group consisting of polylactide, poylglycolide, polysaccharides, proteins, polyesters, polyhydroxyalkanoates, polyalkelene esters, polyamides, polycaprolactone, polyvinyl esters, polyamide esters, polyvinyl alcohols, modified derivatives of caprolactone polymers, polytrimethylene carbonate, polyacrylates, polyethylene glycol, hydrogels, photo-curable hydrogels, terminal diols, and combinations thereof.

In one embodiment, the degradation time is less than 9 months. In another embodiment, the degradation time is less than 6 months.

In one embodiment, the bioactive agent is selected from the group consisting of anti-proliferatives, estrogens, chaperone inhibitors, protease inhibitors, protein-tyrosine kinase inhibitors, leptomycin B, peroxisome proliferator-activated receptor gamma ligands (PPARγ), hypothemycin, nitric oxide, bisphosphonates, epidermal growth factor inhibitors, antibodies, proteasome inhibitors, antibiotics, anti-inflammatories, anti-sense nucleotides, transforming nucleic acids, sirolimus (rapamycin), tacrolimus (FK506), everolimus (certican), temsirolimus (CCI-779) and zotarolimus (ABT-578).

Also described herein is a method of providing a coating with a pre-selected degredation time for a non-erodable stent comprising the steps of: (a) selecting a first bioabsorbable polymer with a first weight average molecular weight; (b) selecting at least one additional bioabsorbable polymer with a second weight average molecular weight; (c) blending said first polymer and said at least one additional polymer thereby forming a polymer blend; (d) optionally associating a bioactive agent with said polymer blend; and (e) coating said polymer blend on a non-erodable metal stent, thereby providing a bioactive coating on said stent with a pre-selected degradation time.

In one embodiment, the first polymer and said at least one additional polymer are selected from the group consisting of polylactide, poylglycolide, polysaccharides, proteins, polyesters, polyhydroxyalkanoates, polyalkelene esters, polyamides, polycaprolactone, polyvinyl esters, polyamide esters, polyvinyl alcohols, modified derivatives of caprolactone polymers, polytrimethylene carbonate, polyacrylates, polyethylene glycol, hydrogels, photo-curable hydrogels, terminal diols, and combinations thereof. In one embodiment, the non-erodable stent comprises metals selected from the group consisting of stainless steel, tantalum, titanium, nickel-titanium alloys, shape memory alloys, super elastic alloys, low-modulus Ti-Nb-Zr alloys, cobalt-nickel alloy steel (MP-35N), and combinations thereof.

In one embodiment, the bioactive agent is dispersed within said polymer blend. In one embodiment, the bioactive agent is selected from the group consisting of anti-proliferatives, estrogens, chaperone inhibitors, protease inhibitors, protein-tyrosine kinase inhibitors, leptomycin B, peroxisome proliferator-activated receptor gamma ligands (PPARγ), hypothemycin, nitric oxide, bisphosphonates, epidermal growth factor inhibitors, antibodies, proteasome inhibitors, antibiotics, anti-inflammatories, anti-sense nucleotides, transforming nucleic acids, sirolimus (rapamycin), tacrolimus (FK506), everolimus (certican), temsirolimus (CCI-779) and zotarolimus (ABT-578).

In one embodiment, the degradation time is less than 9 months. In another embodiment, the degradation time is less than 6 months.

### BRIEF DESCRIPTION OF THE DRAWINGS

**Figure 1** depicts polymer degradation time over the course of 115 days. Two separate polymers are depicted along with a blend of the two polymers.
**Figure 2** depicts polymer degradation time of several polymers and polymer blends over the course of 115 days.
**Figure 3** depicts polymer degradation time as a function of percent mass remaining of the original polymer coating.

### DEFINTIONS

Bioactive Agent: As used herein "bioactive agent" shall include any drug, pharmaceutical compound or molecule having a therapeutic effect in an animal. The use of drug herein falls within the scope of bioactive agent. Exemplary, non-limiting examples include anti-proliferatives including, but not limited to, macrolide antibiotics including FKBP 12 binding compounds, estrogens, chaperone inhibitors, protease inhibitors, protein-tyrosine kinase inhibitors, leptomycin B, peroxisome proliferator-activated receptor gamma ligands (PPARγ), hypothemycin, nitric oxide, bisphosphonates, epidermal growth factor inhibitors, antibodies, proteasome inhibitors, antibiotics, anti-inflammatories, anti-sense nucleotides, and transforming nucleic acids. Bioactive agents can also include cytostatic compounds, chemotherapeutic agents, analgesics, statins, nucleic acids, polypeptides, growth factors, and delivery vectors including, but not limited to, recombinant microorganisms, and liposomes.

Exemplary FKBP 12 binding compounds include sirolimus (rapamycin), tacrolimus (FK506), everolimus (certican or RAD-001), temsirolimus (CCI-779 or amorphous rapamycin 42-ester with 3-hydroxy-2-(hydroxymethyl)-2-methylpropionic acid) and zotarolimus (ABT-578). Additionally, and other rapamycin hydroxyesters may be used in combination with the terpolymers of the present invention.

Biocompatible: As used herein "biocompatible" shall mean any material that does not cause injury or death to the animal or induce an adverse reaction in an animal when placed in intimate contact with the animal's tissues. Adverse reactions include inflammation, infection, fibrotic tissue formation, cell death, or thrombosis.

Biodegradable: As used herein "biodegradable" refers to a polymeric composition that is biocompatible and subject to being broken down *in vivo* through the action of normal biochemical pathways. From time-to-time bioresorbable and biodegradable may be used interchangeably, however they are not coextensive. Biodegradable polymers may or may not be reabsorbed into surrounding tissues, however, all bioresorbable polymers are considered biodegradable. Biodegradable polymers are capable of being cleaved into biocompatible byproducts through chemical- or enzyme-catalyzed hydrolysis.

Nonbiodegradable: As used herein "nonbiodegradable" refers to a polymeric composition that is biocompatible and not subject to being broken down *in vivo* through the action of normal biochemical pathways.

Not Substantially Toxic: As used herein "not substantially toxic" shall mean systemic or localized toxicity wherein the benefit to the recipient is out-weighted by the physiologically harmful effects of the treatment as determined by physicians and pharmacologists having ordinary skill in the art of toxicity.

Pharmaceutically Acceptable: As used herein "pharmaceutically acceptable" refers to all derivatives and salts that are not substantially toxic at effective levels *in vivo.*

### DETAILED DESCRIPTION OF THE INVENTION

Described herein is a polymer system and method for reducing the risk of late thrombosis associated with Drug eluting stent (DES) therapy. A biodegradable polymer-coated stent can be a solution to this problem. Ideally, a stent can be implanted into an appropriate site in the vasculature wherein a bioabsorbable polymer coating can aid in the local delivery of one or more bioactive agents. After the polymer coating has fulfilled its requirement of local delivery of a bioactive agent, it would be advantageous for the coating to be absorbed into the surrounding tissue or expelled from the patient leaving behind the bare metal stent structure.

In one embodiment, stents may be used as a bioactive agent delivery platform. The stents may be vascular stents, urethral stents, biliary stents, or stents intended for use in other ducts and organ lumens. Vascular stents, for example, may be used in peripheral, cerebrovascular, or coronary artery applications. The stents may be rigid expandable stents or pliable self-expanding stents. Any biocompatible metal may be used to fabricate stents. In one embodiment, vascular stents are implanted into coronary arteries immediately following angioplasty. In another embodiment, vascular stents are implanted into the abdominal aorta to treat an abdominal aneurysm.

In one embodiment, the stent is a vascular stent with a cylindrical (tubular) shape. The shape can be defined by a longitudinal axis with a proximal end and a distal end. In addition, the stent has an inner surface which can contact the fluids flowing through the vessel of implantation and an outer surface which contacts the surface of the vessel in which the stent is deployed.

In one embodiment, the stents can be coated with a biodegradable polymer. One possible polymer coating can be a polymer blend. Described herein are polymer blends useful in tailoring bioabsorbtion time by blending polymers with different weight average molecular weights (M_{w}) and/or number average molecular weight (Mₙ). In one embodiment, the bioabsorbtion time is increased, in another embodiment, the bioabsorbtion time is decreased.

In one embodiment, polymeric coatings can be applied to at least a portion of a stent. Suitable bioabsorbable polymers include, but are not limited to, polylactide, poylglycolide, polysaccharides, proteins, polyesters, polyhydroxyalkanoates, polyalkelene esters, polyamides, polycaprolactone, polyvinyl esters, polyamide esters, polyvinyl alcohols, modified derivatives of caprolactone polymers, polytrimethylene carbonate, polyacrylates, polyethylene glycol, hydrogels, photo-curable hydrogels, and terminal diols. The above polymers are generally well received by the vasculature. However, the polymers tend to be hydrophobic and therefore have longer biodegration times as a result.

In one embodiment, a least two polymers are blended to form a polymer system. The polymer system can consist of polymers with different M_{w}. Generally, polymers mentioned above with larger M_{w} have fewer end groups per polymer unit and tend to have large tensile strengths. The polymers with lower M_{w} can have more end groups per polymer unit, more free volumes within the polymer chains, and have more strain strength. The biodegradation time for the low M_{w} equivalent polymers is faster as a result of the increased number of end groups per molecular weight and the ease of water penetration into the polymer chains. It is understood that different polymer backbones have different degradation times, but identical polymer backbones with polymer units of different M_{w} can have different biodegradation times as well.

As a result, generally, a blend of larger M_{w} polymer and lower M_{w} polymer can yield a blend with a tailored biodegradation rate depending on the amount of each polymer added to the blend. In addition, in one embodiment, the higher M_{w} polymers will contribute more tensile strength in the coating and the low M_{w} polymers will enhance the strain strength. Therefore, in addition to tailoring the biodegradation time, the mechanical properties can be tailored as well.

In addition to the benefits of a tailorable biodegradation time, the polymer blends described herein will have a characteristic polydispersity index (PDI). In one embodiment, using one polymer with a low M_{w} and a second polymer with a high M_{w}, the polymer blends will have a wide PDI. Generally, the wider the PDI, the more mechanical strength distribution the polymer blend will have. Therefore, if the PDI is very wide, for example 3, the polymer blend can have exceptional mechanical strength both in tensile and strain. On the other hand, if the PDI is very narrow, for example 1.5, the polymer blend can have reduced mechanical strength distribution either in tensile or strain.

Degradation times for some bare metal stents can be about one month. In one embodiment, the degradation time can be increased thereby creating a new degradation time. In one embodiment, the new degradation time is less than 6 months. In another embodiment, the new degradation time is less than 9 months. In another embodiment, the new degradation time is between about 1 month and about 3 months. In another embodiment, the new degradation time is between about 1 month and about 6 months. In another embodiment, the new degradation time is between about 1 month and about 9 months. In another embodiment, the new degradation time is between about 3 months and about 9 months. In another embodiment, the new degradation time is between about 6 months and about 9 months.

In addition to the tailored degradation time of the polymer coating, the polymer coating can accommodate one or more bioactive agents. The choice of bioactive agent to incorporate, or how much to incorporate, will have a great deal to do with the polymer selected to coat or form the implantable medical device. A person skilled in the art will appreciate that hydrophobic agents prefer hydrophobic polymers and hydrophilic agents prefer hydrophilic polymers. Therefore, coatings and medical devices can be designed for agent or agent combinations with immediate release, sustained release or a combination of the two.

In one embodiment, a polymer blend with hydrophobic regions and hydrophilic regions can be used. For example, a polymer blend wherein the hydrophilic polymers associate themselves on the surface and the hydrophobic polymers assimilate in the core of the polymer blend. In such a case, a hydrophobic bioactive agent can be dispersed in the hydrophobic core of the polymer blend.

Exemplary, non limiting examples of bioactive agents include anti-proliferatives including, but not limited to, macrolide antibiotics including FKBP-12 binding compounds, estrogens, chaperone inhibitors, protease inhibitors, protein-tyrosine kinase inhibitors, leptomycin B, peroxisome proliferator-activated receptor gamma ligands (PPARy), hypothemycin, nitric oxide, bisphosphonates, epidermal growth factor inhibitors, antibodies, proteasome inhibitors, antibiotics, anti-inflammatories, anti-sense nucleotides and transforming nucleic acids. Bioactive agents can also refer to bioactive agents including anti-proliferative compounds, cytostatic compounds, toxic compounds, anti-inflammatory compounds, chemotherapeutic agents, analgesics, antibiotics, protease inhibitors, statins, nucleic acids, polypeptides, growth factors and delivery vectors including recombinant microorganisms, liposomes, and the like.

Exemplary FKBP-12 binding agents include sirolimus (rapamycin), tacrolimus (FK506), everolimus (certican or RAD-001), temsirolimus (CCI-779 or amorphous rapamycin 42-ester with 3-hydroxy-2-(hydroxymethyl)-2-methylpropionic acid as disclosed in USPASN 10/930,487) and zotarolimus (ABT-578; see USPNs 6,015,815 and 6,329,386). Additionally, other rapamycin hydroxyesters as disclosed in USPN 5,362,718 may be used in combination with the polymers described herein.

The polymer coatings discussed herein may be designed with a specific dose of bioactive agent. That dose may be a specific weight of each bioactive agent added, or a ratio of each bioactive agent to polymer. In one embodiment, the medical device can be loaded 0 to 1000 µg of a bioactive agent; in another embodiment, 5 to 500 µg of a bioactive agent; in another embodiment 10 to 250 µg of a bioactive agent; in another embodiment, 15 to 150 µg of a bioactive agent.

A ratio may also be established describing how much bioactive agent is added to the polymer coated on the medical device. In one embodiment, a ratio of 1 part bioactive agent to 1 part polymer may be used; in another embodiment, 1:1-5; in another embodiment, 1:1-9; in another embodiment, 1:1-20. One skilled in the art will appreciate that there are countless combinations that can be contemplated and are all considered to be within the scope of the present description.

In one embodiment, at least a portion of the stent can be coated. In another embodiment, different portions of the stent can be coated with different polymer blends. For example, the external longitudinal body of the stent can be coated with a polymer blend with a very wide PDI thereby providing high mechanical strength to the stent onto which it is coated. In another embodiment, for example, the longitudinal body can be coated as above, however, in addition, the proximal and distal ends can be coated with a polymer having a narrower PDI thereby providing a low mechanical strength to the ends of the stent.

### Example 1

### Synthesis of Copolymers of TMC/DLLA

20 g of trimethylene carbonate (TMC), 30 g of dl-lactide (3,6-dimethyl-1, 4 -dioxane-2, 5-dione) (DLLA), 50 mg of 1,8 octanediol (initiator) and 50 mg of tin(II)2-ethylhexanoate were weighed into a 250 mL three neck flask. The three necks were capped as follows: one neck was fitted with a mechanical stirrer, one neck was fitted with a thermometer, and one neck was fitted with a condenser coupled to a nitrogen bubbler. The flask was purged with nitrogen throughout the reaction. The reactant was stirred with a Teflon® blade at 110 rpm. The flask was then placed in a 150°C silicon oil bath with a stir bar mixing the reactants for 24 hours. When the reaction was ended, the reactant was dissolved in 200 mL of chloroform and poured into 800 mL of methanol for precipitation at room temperature. This procedure was repeated three times. The final purified polymer was dissolved into chloroform and poured into a PTFE tray. The tray was placed in a vacuum oven over night at 50°C.

Different weight polymers were generated using different amounts of 1,8-octanediol (initator). Some exemplary polymers are shown in Table 1 below.

**Table 1**

| **Polymer** | **Monomers** | **NMR Proton Ratio** | **Mₙ** | **M_{w}** | **PDI** | **Tg (°C)** |
|---|---|---|---|---|---|---|
| 1 | TMC/DLLA | 19.72/80.28 | 13528 | 20373 | 1.49 | 30.66 |
| 2 | TMC/DLLA | 32.07/67.93 | 18162 | 27830 | 1.53 | 20.52 |
| 3 | TMC/DLLA | 32.05/67.95 | 28900 | 47667 | 1.65 | 23.07 |
| 4 | TMC/DLLA | 10.58/89.42 | 39818 | 62250 | 1.56 | 36.17 |
| 5 | TMC/DLLA | 34.37/65.63 | 54798 | 91292 | 1.67 | 22.14 |
| 6 | TMC/DLLA | 33.90/66.10 | 91337 | 167485 | 1.84 | 23.61 |
| 7 | TMC/DLLA | 21.72/78.28 | 161156 | 303624 | 1.89 | 25.95 |
| 8* | TMC/DLLA | - | 23709 | 41459 | 1.75 | 26.63 |
| 9** | TMC/DLLA | - | 32481 | 90420 | 2.78 | 20.63 |
| 10*** | TMC/DLLA | - | 30688 | 75150 | 2.45 | 25.04 |

| | | | | | | |
|---|---|---|---|---|---|---|
| *Polymer 8 was a 50:50 weight mixture of Polymers 2 and 4. **Polymer 9 was a 50:50 weight mixture of Polymers 2 and 6. ***Polymer 10 was a 33:33:33 weight mixture of Polymers 2, 4 and 6. | | | | | | |

### Example 2

### Synthesis of Terpolymers of TMC/PEG/DLLA

20 g of TMC, 30 g of DLLA, 2.5 gram of polyethylene glycol (PEG) (molecular weight 3500), and 50 mg of tin(II)2-ethylhexanoate were weighed into a 250 mL three neck flask. The three necks were capped as follows: one neck was fitted with a mechanical stirrer, one neck was fitted with a thermometer, and one neck was fitted with a condenser coupled to a nitrogen bubbler. The flask was purged with nitrogen throughout the reaction. The reactant was stirred with a Teflon® blade at 110 rpm. The flask was then placed in a 150°C silicon oil bath with a stir bar mixing the reactants for 16 hours. When the reaction was ended, the reactant was dissolved in 200 mL of chloroform and poured into 800 mL of methanol to precipitate at room temperature. This procedure was repeated three times. The final purified polymer was dissolved into chloroform and poured into a PTFE tray. The tray was placed in a vacuum oven over night at 50°C.

Some exemplary polymers made according to this method are shown in Table 2 below.

**Table 2**

| **Polymer** | **Feed Ratio (wt)** | **NMR Proton Ratio** | **Mₙ** | **M_{w}** | **PDI** | **Tg (°C)** |
|---|---|---|---|---|---|---|
| 11 | PEG/TMC/DLLA 2.5/20/30 | 0.12/33.68/66.07 | 52210 | 82141 | 1.58 | 12.51 |
| 12 | PEG/TMC/DLLA | 0.12/33.28/66.48 | 50580 | 80421 | 1.60 | 14.37 |
| | 2.5/20/30 | | | | | |
| 13 | PEG/TMC/DLLA | 0.12/33.88/65.87 | 65935 | 98661 | 1.50 | 12.93 |
| | 2.5/20/30 | | | | | |

### Example 3

### Synthesis of Terpolymers of TMC/DLLA/GA

15 g of TMC, 17.5 g of DLLA, 17.5 g of glycolide (GA), and 50 mg of tin(II)2-ethylhexanoate were weighed into a 250 mL three neck flask. The three necks were capped as follows: one neck was fitted with a mechanical stirrer, one neck was fitted with a thermometer, and one neck was fitted with a condenser coupled to a nitrogen bubbler. The flask was purged with nitrogen throughout the reaction. The reactant was stirred with a Teflon® blade at 110 rpm. The flask was then placed in a 150°C silicon oil bath with a stir bar mixing the reactants for 16 hours. When the reaction was ended, the reactant was dissolved in 200 mL of chloroform and poured into 800 mL of methanol to precipitate at room temperature. This procedure was repeated three times. The final purified polymer was dissolved into chloroform and poured into a PTFE tray. The tray was placed in a vacuum oven over night at 50°C.

Some exemplary polymers made according to this method are shown in Table 3 below.

**Table 3**

| **Polymer** | **Feed Ratio (wt)** | **NMR Proton Ratio** | **Mₙ** | **M_{w}** | **PDI** | **T_{g} (°C)** |
|---|---|---|---|---|---|---|
| 14 | TMC/DLLA/GA | 22.06/31.21/46.73 | 11705 | 18388 | 1.57 | 18.81 |
| | 15/17.5/17.5 | | | | | |
| 15 | TMC/DLLA/GA | 21.86/32.73/45.41 | 48997 | 78513 | 1.60 | 20.43 |
| | 15/17.5/17.5 | | | | | |
| 16 | TMC/DLLA/GA | 21.47/33.51/45.02 | 61685 | 96827 | 1.57 | 19.54 |
| | 15/17.5/17.5 | | | | | |
| 17 | TMC/DLLA/GA | 20.01/33.54/46.45 | 94216 | 149952 | 1.59 | 28.13 |
| | 15/17.5/17.5 | | | | | |

### Example 4

### Polymer Degradation Time

Several of the polymers produced according to Example 1 were tested *in vitro.* Their respective degradation times were measured both as a function of molecular weight and a function of percent mass remaining. Polymers used in this example may have different molecular weights than polymers listed in Table 1, but were made in a similar manner and are therefore numbered accordingly as a reference.

Samples were prepared and tested according to the following procedure: A_stainless steel NP35 sheet was cut into 3 x 1 inch rectangular coupons and cleaned with methylene chloride in an ultrasonic bath. The cleaned coupons were dried in a vacuum oven. Next, two polymers with different molecular weights, one high and one low, were weighted out. These polymers were dissolved in methylene chloride at 5% w/v. The polymer solutions were placed on a shaker for 30 minutes. Then, equal volumes of these two polymer solutions were extracted and mixed in a pre-cleaned glass bottle. The cleaned coupons were dipped into the polymer solutions and dried using a heating gun. The coated coupons were further dried in a vacuum oven. The dried coupons were then weighted using a balance. The target coating weight was 24 mg per coupon (equal to 0.6 mg per polymer on per 18mm stent). The coated coupons were placed in test tubes filled with phosphate buffered saline (PBS). The test tubes were placed in an isothermal incubator at 37°C. Three coupons were prepared for each time point. The coupons were taken out at 2, 4, 8, 12 and 16 week intervals to dry by heating gun first, then by vacuum oven. The dried coupons were weighed on balance to get remaining weight then washed with tetrahydrofuran (THF). The THF solution was then subjected to gel permeation chromatography to measure the molecular weight of the polymer in the THF solution.

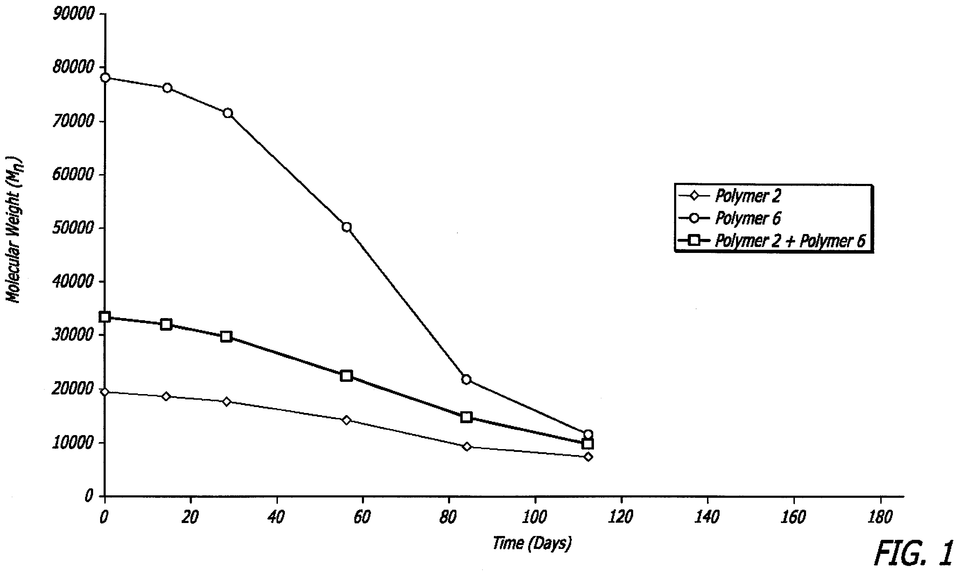

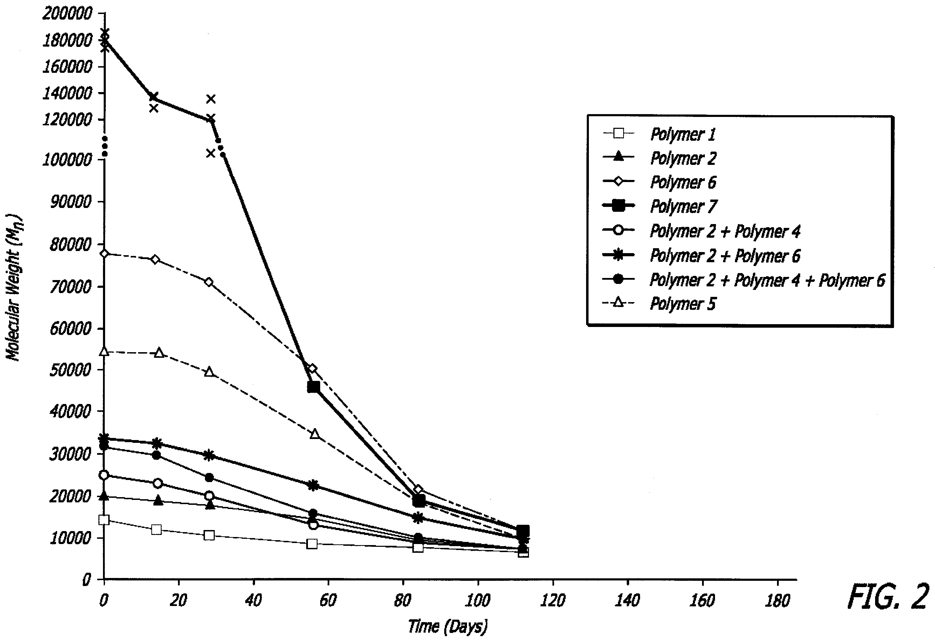

The results indicated that mixing of a higher molecular weight polymer and a lower molecular weight polymer results in a polymer blend with an intermediate molecular weight and percent mass lost. Referring to Figure 1, polymer 6 has a high Mₙ and polymer 2 has a much lower Mₙ than polymer 6. Blending 50/50 (weight/weight) of polymer 2 and polymer 6 gives a polymer system with a Mₙ that is lower than that of polymer 6 but higher than polymer 2. One skilled in the art can appreciate that mixing more or less of one polymer in the system will allow the Mₙ of the system to be fine tuned. Figure 2 shows the degradation of several polymer systems described herein.

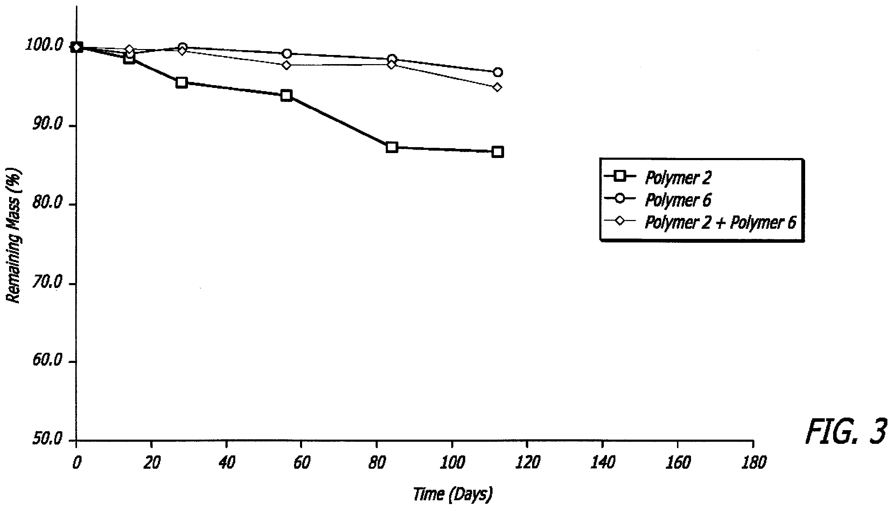

Figure 3 shows the percent mass lost from a polymer system over time. Polymer 6 with a higher Mₙ has a greater percent mass remaining after the 115 day test period than polymer 2 with a lower Mₙ. By blending 50/50 of polymer 2 and polymer 6, the percent mass remaining is lowered as compared to polymer 6 alone. One skilled in the art can appreciate that mixing more or less of one polymer in the system will allow the percent mass lost (degradation time) of the system to be fine tuned. For example, adding more of polymer 2 and less of polymer 6 will result in a polymer system with a faster degradation time than a 50/50 blend. On the other hand, a blend of more polymer 6 and less polymer 2 will result in a polymer system with a longer degradation time as compared to the 50/50 blend.

### Example 5

### Metal Stent Cleaning Procedure

Stainless steel stents were placed a glass beaker and covered with reagent grade or better hexane. The beaker containing the hexane immersed stents was then placed into an ultrasonic water bath and treated for 15 minutes at a frequency of between approximately 25 to 50 KHz. Next the stents were removed from the hexane and the hexane was discarded. The stents were then immersed in reagent grade or better 2-propanol and vessel containing the stents and the 2-propanol was treated in an ultrasonic water bath as before. Following cleaning the stents with organic solvents, they were thoroughly washed with distilled water and thereafter immersed in 1.0 N sodium hydroxide solution and treated at in an ultrasonic water bath as before. Finally, the stents were removed from the sodium hydroxide, thoroughly rinsed in distilled water and then dried in a vacuum oven over night at 40°C. After cooling the dried stents to room temperature in a desiccated environment they were weighed their weights were recorded.

### EXAMPLE 6

### Coating a Clean. Dried Stent Using a Bioactive Agent/Polymer System

In a non-limiting example, the bioactive agent is zotarolimus and the polymer system is Polymer 2 from the examples above. Both the polymer and zotarolimus are dissolved in an appropriate solvent. Persons having ordinary skill in the art of polymer chemistry can easily pair the appropriate solvent system to the polymer-drug combination and achieve optimum results with no more than routine experimentation.

Zotarolimus is carefully weighed and added to a small neck glass bottle containing solvent. The zotarolimus suspension is then thoroughly mixed until a clear solution is achieved.

Next Polymer 2 is added to the zotarolimus solution and mixed until Polymer 2 dissolves forming a bioactive agent/polymer solution.

The cleaned, dried stents are coated using either spraying techniques or dipped into the drug/polymer solution. The stents are coated as necessary to achieve a final coating weight of between approximately 10 µg to 1 mg. Finally, the coated stents are dried in a vacuum oven at 50°C overnight. The dried, coated stents are weighed and the weights recorded.

The concentration of bioactive agent loaded onto (into) the stents is determined based on the final coating weight. Final coating weight is calculated by subtracting the stent's pre-coating weight from the weight of the dried, coated stent.

At the very least, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Moreover, any combination of the above-described elements in all possible variations thereof is encompassed by the invention unless otherwise indicated herein or otherwise clearly contradicted by context.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. A medical device comprising:
(a) a stent comprising a non-erodable metal;
(b) a bioabsorbable polymer system coated on at least a portion of said metal; said polymer system comprising a blend of at least two polymers having different weight average molecular weights; wherein the ratio of weight average molecular weights is 1:2 to 1:10 and provides a pre-selected bioabsorption time; and
(c) at least one bioactive agent dispersed in at least a portion of said polymer coating.

2. The medical device system according to claim 1 wherein said stent is selected from the group consisting of woven stents, individual ring stents, sequential ring stents, closed cell stents, open cell stents, laser cut tube stents, ratchet stents, and modular stents.

3. The medical device system according to claim 1 wherein said metal is selected from the group consisting of stainless steel, tantalum, titanium, nickel-titanium alloys, shape memory alloys, super elastic alloys, low-modulus Ti-Nb-Zr alloys, cobalt-nickel alloy steel (MP-35N), and combinations thereof.

4. The medical device system according to claim 1 wherein said polymers are selected from the group consisting of polylactide, poylglycolide, polysaccharides, proteins, polyesters, polyhydroxyalkanoates, polyalkelene esters, polyamides, polycaprolactone, polyvinyl esters, polyamide esters, polyvinyl alcohols, modified derivatives of caprolactone polymers, polytrimethylene carbonate, polyacrylates, polyethylene glycol, hydrogels, photo-curable hydrogels, terminal diols, and combinations thereof.

5. The medical device system according to claim 1 wherein said degradation time is less than 9 months.

6. The medical device system according to claim 1 wherein said degradation time is less than 6 months.

7. The medical device system according to claim 1 wherein said bioactive agent is selected from the group consisting of anti-proliferatives, estrogens, chaperone inhibitors, protease inhibitors, protein-tyrosine kinase inhibitors, leptomycin B, peroxisome proliferator-activated receptor gamma ligands (PPAHγ), hypothemycin, nitric oxide, bisphosphonates, epidermal growth factor inhibitors, antibodies, proteasome inhibitors, antibiotics, anti-inflammatories, anti-sense nucleotides, transforming nucleic acids, sirolimus (rapamycin), tacrolimus (FK506), everolimus (certican), temsirolimus (CCI-779) and zotarolimus (ABT-578).

8. A method of providing a coating with a pre-selected degradation time for a non-erodable stent, the method comprising the steps of:
(a) selecting a first bioabsorbable polymer with a first weight average molecular weight;
(b) selecting at least one additional bioabsorbable polymer with a second weight average molecular weight;
(c) blending said first polymer and said at least one additional polymer thereby forming a polymer blend, wherein said polymer blend comprises a weight average molecular weight ratio, wherein said weight average molecular weight ratio is 1:2 to 1:10;
(d) optionally associating a bioactive agent with said polymer blend; and
(e) coating said polymer blend on a non-erodable metal stent, thereby providing a bioactive coating on said stent with a pre-selected degradation time.

9. The method according to claim 8 wherein said first polymer and said at least one additional polymer are selected from the group consisting of polylactide, poylglycolide, polysaccharides, proteins, polyesters, polyhydroxyalkanoates, polyalkelene esters, polyamides, polycaprolactone, polyvinyl esters, polyamide esters, polyvinyl alcohols, modified derivatives of caprolactone polymers, polytrimethylene carbonate, polyacrylates, polyethylene glycol, hydrogels, photo-curable hydrogels, terminal diols, and combinations thereof.

10. The method according to claim 8 wherein said non-erodable stent comprises metals selected from the group consisting of stainless steel, tantalum, titanium, nickel-titanium alloys, shape memory alloys, super elastic alloys, low-modulus Ti-Nb-Zr alloys, cobalt-nickel alloy steel (MP-35N), and combinations thereof.

11. The method according to claim 8 wherein said bioactive agent is dispersed within said polymer blend.

12. The method according to claim 8 wherein said bioactive agent is selected from the group consisting of anti-proliferatives, estrogens, chaperone inhibitors, protease inhibitors, protein-tyrosine kinase inhibitors, leptomycin B, peroxisome proliferator-activated receptor gamma ligands (PPAHγ), hypothemycin, nitric oxide, bisphosphonates, epidermal growth factor inhibitors, antibodies, proteasome inhibitors, antibiotics, anti-inflammatories, anti-sense nucleotides, transforming nucleic acids, sirolimus (rapamycin), tacrolimus (FK506), everolimus (certican), temsirolimus (CCI-779) and zotarolimus (ABT-578).

13. The method according to claim 8 wherein said degradation time is less than 9 months.

14. The method according to claim 8 wherein said degradation time is less than 6 months.

## Patentansprüche

1. Medizinische Vorrichtung, umfassend:
(a) einen Stent, umfassend ein nicht erodierbares Metall;
(b) ein bioabsorbierbares Polymersystem, mit dem mindestens ein Teil des Metalls beschichtet ist; wobei das Polymersystem eine Mischung aus mindestens zwei Polymeren mit unterschiedlichen gewichtsmittleren Molekulargewichten umfasst; wobei das Verhältnis der gewichtsmittleren Molekulargewichte 1:2 bis 1:10 beträgt und eine vorgewählte Bioabsorptionszeit bereitstellt; und
(c) mindestens ein bioaktives Mittel, das in mindestens einem Teil der Polymerbeschichtung verteilt ist.

2. Medizinisches Vorrichtungssystem nach Anspruch 1, wobei der Stent aus der Gruppe bestehend aus gewebten Stents, individuellen Ringstents, sequentiellen Ringstents, geschlossenzelligen Stents, offenzelligen Stents, lasergeschnittenen Röhrenstents, Ratschenstents und modularen Stents ausgewählt ist.

3. Medizinisches Vorrichtungssystem nach Anspruch 1, wobei das Metall aus der Gruppe bestehend aus Edelstahl, Tantal, Titan, Nickel-Titan-Legierungen, Formgedächtnislegierungen, superelastischen Legierungen, Ti-Nb-Zr-Legierungen mit niedrigem Modul, Kobalt-Nickel-Legierungsstahl (MP-35N) und Kombinationen davon ausgewählt ist.

4. Medizinisches Vorrichtungssystem nach Anspruch 1, wobei die Polymere aus der Gruppe bestehend aus Polylactid, Polyglycolid, Polysacchariden, Proteinen, Polyestern, Polyhydroxyalkanoaten, Polyalkylenestern, Polyamiden, Polycaprolacton, Polyvinylestern, Polyamidestern, Polyvinylalkoholen, modifizierten Derivaten von Caprolactonpolymeren, Polytrimethylencarbonat, Polyacrylaten, Polyethylenglycol, Hydrogelen, fotohärtbaren Hydrogelen, endständigen Diolen und Kombinationen davon ausgewählt sind.

5. Medizinisches Vorrichtungssystem nach Anspruch 1, wobei die Abbauzeit weniger als 9 Monate beträgt.

6. Medizinisches Vorrichtungssystem nach Anspruch 1, wobei die Abbauzeit weniger als 6 Monate beträgt.

7. Medizinisches Vorrichtungssystem nach Anspruch 1, wobei das bioaktive Mittel aus der Gruppe bestehend aus Antiproliferativa, Östrogenen, Chaperon-Inhibitoren, Protease-Inhibitoren, Protein-Tyrosinkinase-Inhibitoren, Leptomycin B, Peroxisomproliferator-aktivierten Rezeptor-gamma-Liganden (PPAHγ), Hypothemycin, Stickstoffmonoxid, Bisphosphonaten, Inhibitoren des epidermalen Wachstumsfaktors, Antikörpern, Proteasom-Inhibitoren, Antibiotika, Entzündungshemmern, Antisense-Nukleotiden, transformierenden Nukleinsäuren, Sirolimus (Rapamycin), Tacrolimus (FK506), Everolimus (Certican), Temsirolimus (CCI-779) und Zotarolimus (ABT-578) ausgewählt ist.

8. Verfahren zum Bereitstellen einer Beschichtung mit einer vorgewählten Abbauzeit für einen nicht erodierbaren Stent, wobei das Verfahren die folgenden Schritte umfasst:
(a) Auswählen eines ersten bioabsorbierbaren Polymers mit einem ersten gewichtsmittleren Molekulargewicht;
(b) Auswählen von mindestens einem zusätzlichen bioabsorbierbaren Polymer mit einem zweiten gewichtsmittleren Molekulargewicht;
(c) Mischen des ersten Polymers und des mindestens einen zusätzlichen Polymers, wodurch eine Polymermischung gebildet wird, wobei die Polymermischung ein Verhältnis der gewichtsmittleren Molekulargewichte umfasst, wobei das Verhältnis der gewichtsmittleren Molekulargewichte 1:2 bis 1:10 beträgt;
(d) wahlweise Assoziieren eines bioaktiven Mittels mit der Polymermischung; und
(e) Beschichten eines nicht erodierbaren Metallstents mit der Polymermischung, wodurch eine bioaktive Beschichtung auf dem Stent mit einer vorgewählten Abbauzeit bereitgestellt wird.

9. Verfahren nach Anspruch 8, wobei das erste Polymer und das mindestens eine zusätzliche Polymer aus der Gruppe bestehend aus Polylactid, Polyglycolid, Polysacchariden, Proteinen, Polyestern, Polyhydroxyalkanoaten, Polyalkylenestern, Polyamiden, Polycaprolacton, Polyvinylestern, Polyamidestern, Polyvinylalkoholen, modifizierten Derivaten von Caprolactonpolymeren, Polytrimethylencarbonat, Polyacrylaten, Polyethylenglycol, Hydrogelen, fotohärtbaren Hydrogelen, endständigen Diolen und Kombinationen davon ausgewählt sind.

10. Verfahren nach Anspruch 8, wobei der nicht erodierbare Stent Metalle umfasst, die aus der Gruppe bestehend aus Edelstahl, Tantal, Titan, Nickel-Titan-Legierungen, Formgedächtnislegierungen, superelastischen Legierungen, Ti-Nb-Zr-Legierungen mit niedrigem Modul, Kobalt-Nickel-Legierungsstahl (MP-35N) und Kombinationen davon ausgewählt sind.

11. Verfahren nach Anspruch 8, wobei das bioaktive Mittel in der Polymermischung verteilt ist.

12. Verfahren nach Anspruch 8, wobei das bioaktive Mittel aus der Gruppe bestehend aus Antiproliferativa, Östrogenen, Chaperon-Inhibitoren, Protease-Inhibitoren, Protein-Tyrosinkinase-Inhibitoren, Leptomycin B, Peroxisomproliferator-aktivierten Rezeptor-gamma-Liganden (PPAHγ), Hypothemycin, Stickstoffmonoxid, Bisphosphonaten, Inhibitoren des epidermalen Wachstumsfaktors, Antikörpern, Proteasom-Inhibitoren, Antibiotika, Entzündungshemmern, Antisense-Nukleotiden, transformierenden Nukleinsäuren, Sirolimus (Rapamycin), Tacrolimus (FK506), Everolimus (Certican), Temsirolimus (CCI-779) und Zotarolimus (ABT-578) ausgewählt ist.

13. Verfahren nach Anspruch 8, wobei die Abbauzeit weniger als 9 Monate beträgt.

14. Verfahren nach Anspruch 8, wobei die Abbauzeit weniger als 6 Monate beträgt.

## Revendications

1. Dispositif médical comprenant :
(a) un stent comprenant un métal non-érodable ;
(b) un système de polymères bioabsorbables revêtus sur au moins une partie dudit métal ; ledit système de polymères comprenant un mélange d'au moins deux polymères ayant différentes masses moléculaires moyennes en poids ; dans lequel le rapport des masses moléculaires moyennes en poids est compris entre 1:2 et 1:10 et fournit un temps de bioabsorption pré-choisi ; et
(c) au moins un agent bioactif dispersé dans au moins une partie dudit revêtement de polymères.

2. Système de dispositif médical selon la revendication 1, dans lequel ledit stent est choisi dans le groupe constitué par des sents tissés, des stents annulaires individuels, des stents annulaires successifs, des stents à cellules fermées, des stents à cellules ouvertes, des stents à tubes coupés au laser, des stents à cliquets et des stents modulaires.

3. Système de dispositif médical selon la revendication 1, dans lequel ledit métal est choisi dans le groupe constitué par de l'acier inoxydable, du tantale, du titane, des alliages de nickel-titane, des alliages à mémoire de forme, des alliages super élastiques, des alliages de Ti-Nb-Zr à faible module, un acier à base d'alliage de cobalt-nickel (MP-35N) et des combinaisons de ceux-ci.

4. Système de dispositif médical selon la revendication 1, dans lequel lesdits polymères sont choisis dans le groupe constitué par un polylactide, un poylglycolide, des polysaccharides, des protéines, des polyesters, des polyhydroxyalcanoates, des esters de polyalkylènes, des polyamides, une polycaprolactone, des esters polyvinyliques, des esters de polyamides, des alcools polyvinyliques, des dérivés modifiés de polymères de caprolactone, un polycarbonate de triméthylène, des polyacrylates, du polyéthylène glycol, des hydrogels, des hydrogels photo-durcissables, des diols terminaux, et des combinaisons de ceux-ci.

5. Système de dispositif médical selon la revendication 1, dans lequel ledit temps de dégradation est inférieur à 9 mois.

6. Système de dispositif médical selon la revendication 1, dans lequel ledit temps de dégradation est inférieur à 6 mois.

7. Système de dispositif médical selon la revendication 1, dans lequel ledit agent bioactif est choisi dans le groupe constitué par des agents anti-prolifératifs, des oestrogènes, des inhibiteurs de protéines chaperon, des inhibiteurs de protéases, des inhibiteurs de protéines tyrosine kinases, la leptomycine B, des ligands de récepteurs gamma activés par des proliférateurs de peroxysomes (PPARγ), l'hypothémycine, l'oxyde nitrique, des bisphosphonates, des inhibiteurs de facteurs de croissance épidermiques, des anticorps, des inhibiteurs de protéasomes, des antibiotiques, des anti-inflammatoires, des nucléotides antisens, des acides nucléiques transformants, le sirolimus (rapamycine), le tacrolimus (FK506), l'évérolimus (certican), le temsirolimus (CCI-779) et le zotarolimus (ABT-578).

8. Procédé de fourniture d'un revêtement présentant un temps de dégradation pré-choisi pour un stent non-érodable, le procédé comprenant les étapes de :
(a) sélection d'un premier polymère bioabsorbable ayant une première masse moléculaire moyenne en poids ;
(b) sélection d'au moins un polymère bioabsorbable supplémentaire ayant une seconde masse moléculaire moyenne en poids ;
(c) mélange dudit premier polymère et dudit au moins un polymère supplémentaire, ce qui permet de former un mélange de polymères, dans lequel ledit mélange de polymères comprend un rapport de masses moléculaires moyennes en poids, dans lequel ledit rapport de masses moléculaires moyennes en poids est compris entre 1:2 et 1:10 ;
(d) éventuellement association d'un agent bioactif avec ledit mélange de polymères ; et
(e) revêtement dudit mélange de polymères sur un stent en métal non-érodable,
fournissant ainsi sur ledit stent un revêtement bioactif présentant un temps de dégradation pré-choisi.

9. Procédé selon la revendication 8, dans lequel ledit premier polymère et ledit au moins un polymère supplémentaire sont choisis dans le groupe constitué par un polylactide, un poylglycolide, des polysaccharides, des protéines, des polyesters, des polyhydroxyalcanoates, des esters de polyalkylènes, des polyamides, une polycaprolactone, des esters polyvinyliques, des esters de polyamides, des alcools polyvinyliques, des dérivés modifiés de polymères de caprolactone, un polycarbonate de triméthylène, des polyacrylates, du polyéthylène glycol, des hydrogels, des hydrogels photo-durcissables, des diols terminaux, et des combinaisons de ceux-ci.

10. Procédé selon la revendication 8, dans lequel ledit stent non-érodable comprend des métaux choisis dans le groupe constitué par de l'acier inoxydable, du tantale, du titane, des alliages de nickel-titane, des alliages à mémoire de forme, des alliages super élastiques, des alliages de Ti-Nb-Zr à faible module, un acier à base d'alliage de cobalt-nickel (MP-35N) et des combinaisons de ceux-ci.

11. Procédé selon la revendication 8, dans lequel ledit agent bioactif est dispersé dans ledit mélange de polymères.

12. Procédé selon la revendication 8, dans lequel ledit agent bioactif est choisi dans le groupe constitué par des agents anti-prolifératifs, des oestrogènes, des inhibiteurs de protéines chaperon, des inhibiteurs de protéases, des inhibiteurs de protéines tyrosine kinases, la leptomycine B, des ligands de récepteurs gamma activés par des proliférateurs de peroxysomes (PPARy), l'hypothémycine, l'oxyde nitrique, des bisphosphonates, des inhibiteurs de facteurs de croissance épidermiques, des anticorps, des inhibiteurs de protéasomes, des antibiotiques, des anti-inflammatoires, des nucléotides antisens, des acides nucléiques transformants, le sirolimus (rapamycine), le tacrolimus (FK506), l'évérolimus (certican), le temsirolimus (CCI-779) et le zotarolimus (ABT-578).

13. Procédé selon la revendication 8, dans lequel ledit temps de dégradation est inférieur à 9 mois.

14. Procédé selon la revendication 8, dans lequel ledit temps de dégradation est inférieur à 6 mois.
